# EUROPEAN PATENT APPLICATION

(11) **EP 1 745 751 A1**
(43) Date of publication of application: **24.01.2007**
(21) Application number: 06253841.8
(22) Date of filing: 21.07.2006
(51) Int. Cl.: A61B 17/11

(54) **Flexible endoscopic anastomotic ring applier device**

(30) Priority: 22.07.2005 US 187674
(71) Applicant: ETHICON ENDO-SURGERY, INC., Cincinnati, Ohio 45242 (US)
(72) Inventor: Griffith, David B., Cincinnati, OH 45226 (US); Ortiz, Mark S., Milford, OH 45150 (US)
(74) Representative: Tunstall, Christopher Stephen

(57) **Abstract**

A surgical instrument for deploying an anastomotic ring device has a ring deployment mechanism, which is configured to receive and deploy an anastomotic ring. The instrument further comprises a flexible elongate shaft having one or more actuation cables extending therethrough. The shaft also has an imaging element, which is coupled to a camera with a lens located in the tip of the instrument. The instrument may be inserted through the esophagus of a patient to deploy an anastomotic ring device.

## Description

### FIELD OF THE INVENTION

The present invention relates, in general, to surgery and, more particularly, to a device for performing a surgical procedure on the digestive system.

### BACKGROUND OF THE INVENTION

The percentage of the world population suffering from morbid obesity is steadily increasing. Severely obese persons may be susceptible to increased risk of heart disease, stroke, diabetes, pulmonary disease, and accidents. Because of the effects of morbid obesity on the life of the patient, methods of treating morbid obesity have been the subject of intense research.

One known method for treating morbid obesity includes the use of anastomotic rings. Devices for applying anastomotic rings are known in the art. Devices of this nature are commonly adapted to insert a compressed anastomotic ring to an anastomotic opening formed between proximate gastrointestinal tissue walls. These applier devices may utilize a ring deployment mechanism comprising an expansion element that is actuated once the compressed ring is placed in the anastomotic opening, causing the anastomotic ring to expand from its compressed, cylindrically-shaped position to an actuated, hollow rivet-shaped position.

It may be desirable for the surgeon to insert the applier device through the patient's esophagus. Further, it may be desirable for the surgeon to have a view of the anastomosis site. While it is possible to insert an endoscope to view the site of the anastomotic attachment, this may disadvantageously add extra steps and cost to the surgery, require additional space and/or incisions, or introduce other undesired consequences.

### BRIEF SUMMARY OF THE INVENTION

Several embodiments of the present invention provide an anastomotic ring applier device that allows the surgeon to introduce the device transorally and to view the anastomotic attachment site.

In one embodiment, a surgical instrument is operable to deploy an anastomotic ring device at an anastomosis site. The instrument comprises a ring deployment mechanism. The ring deployment mechanism is configured to receive and deploy an anastomotic ring. The instrument further comprises an elongate shaft comprising a plurality of actuation members. Each of the actuation members is operable to communicate one or more actuating forces to the ring deployment mechanism. The shaft is flexible. The instrument further comprises one or more actuators. Each of the one or more actuators is operable to communicate one or more actuating forces to at least one of the actuation members.

In another embodiment, a surgical instrument is operable to deploy an anastomotic ring device at an anastomosis site. The instrument comprises a handle having one or more actuators. Each of the one or more actuators is configured to receive user input to provide one or more actuating forces. The instrument further comprises an elongate shaft having a first end and a second end. The handle is connected to the first end of the elongate shaft. The elongate shaft comprises one or more actuation members. The one or more actuation members are in communication with the one or more actuators. The shaft and the one or more actuation members are flexible. The instrument further comprises a ring deployment mechanism positioned adjacent the second end of the shaft. The ring deployment mechanism is configured to receive an anastomotic ring device. The one or more actuation members are configured to communicate the one or more actuating forces to the ring deployment mechanism. The ring deployment mechanism is operable to deploy the anastomotic ring device in response to at least one of the one or more actuating forces.

In another embodiment, a method for deploying an anastomotic ring device at an anastomosis site comprises providing an instrument to deploy the anastomotic ring device. The instrument comprises a ring deployment mechanism. The ring deployment mechanism is configured to receive and deploy the anastomotic ring device. The instrument further comprises an elongate shaft comprising a plurality of actuation members. Each of the actuation members is operable to communicate one or more actuating forces to the ring deployment mechanism. The instrument further comprises one or more actuators. Each of the one or more actuators is operable to communicate one or more actuating forces to at least one of the actuation members. The method further comprises inserting at least a portion of the instrument through the esophagus of a patient to reach an anastomosis site. The method further comprises deploying the anastomotic ring device at the anastomosis site. The method further comprises withdrawing the at least a portion of the instrument from the esophagus of the patient.

More embodiments will be described below. Other embodiments will be apparent to those of ordinary skill in the art.

### BRIEF DESCRIPTION OF THE FIGURES

The accompanying drawings, which are incorporated in and constitute a part of this specification, illustrate versions of the invention, and, together with the general description of the invention given above, and the detailed description of the versions given below, serve to explain the principles of the present invention.

FIGURE I is a perspective view of an anastomotic ring applier device, shown with a ring deployment mechanism in an unactuated position.

FIGURE 2 is a partial perspective view of the distal portion of an anastomotic ring applier device holding an anastomotic ring in an unactuated position.

FIGURE 3 is a partial perspective view of the distal portion of the device of FIGURE 2 holding an anastomotic ring in the actuated position.

FIGURE 4 is a frontal view of an actuated anastomotic ring.

FIGURE 5 is a perspective view of the device of FIGURE 1, shown with a distal portion of the ring deployment mechanism in a partially actuated position.

FIGURE 6 is a perspective view of the device of FIGURE 1, shown with the distal portion and a proximal portion of the ring deployment mechanism each in a partially actuated position.

FIGURE 7 is a perspective view of the device of FIGURE 1, shown with the distal portion and the proximal portion of the ring deployment mechanism each in a fully actuated position.

FIGURE 8 is an exploded view of a ring deployment mechanism and a visualization system of the device of FIGURE 1.

FIGURE 9 is an exploded view of an actuation mechanism of the device of FIGURE 1.

FIGURE 10 is a partial cross-sectional view of the device of FIGURE 1, shown with the ring deployment mechanism in an unactuated position.

FIGURE 11 is a partial cross-sectional view of the device of FIGURE 1, taken along Plane 11 of FIGURE 10, shown with the ring deployment mechanism in an unactuated position.

FIGURE 12 is a partial cross-sectional view of the device of FIGURE 1, shown with a distal portion of the ring deployment mechanism in a partially actuated position.

FIGURE 13 is a partial cross-sectional view of the device of FIGURE 1, shown with the distal portion and a proximal portion of the ring deployment mechanism each in a partially actuated position.

FIGURE 14 is a partial cross-sectional view of the device of FIGURE 1, shown inserted through an anastomotic opening, with the distal portion and the proximal portion of the ring deployment mechanism each in a fully actuated position.

FIGURE 15 is a cross-sectional view taken at Plane 15 of the device of FIGURE 10.

FIGURE 16 is a cross-sectional view taken at Plane 16 of the device of FIGURE 10.

FIGURE 17 is a cross-sectional view taken at Plane 17 of the device of FIGURE 10.

### DETAILED DESCRIPTION OF EMBODIMENTS OF THE INVENTION

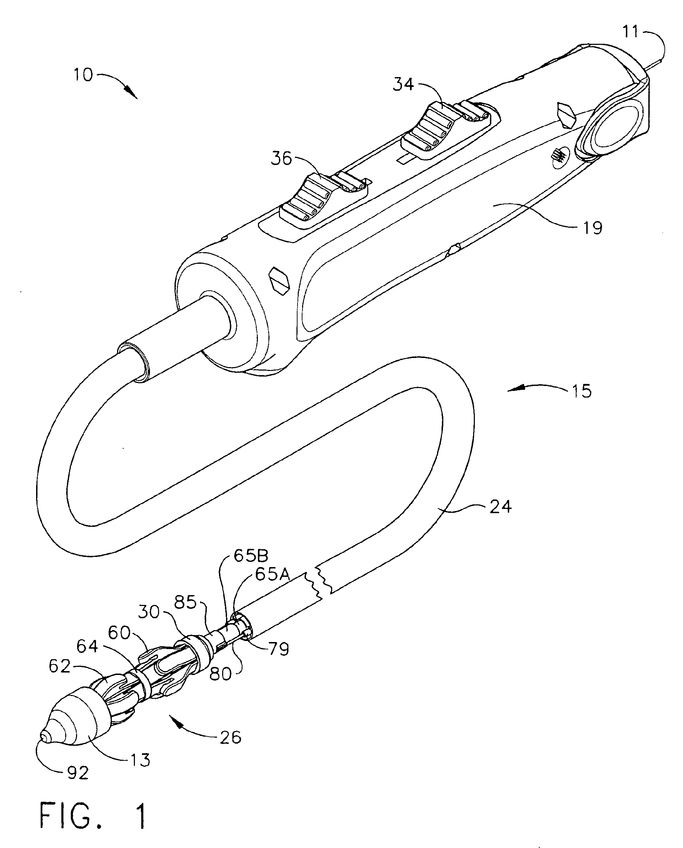

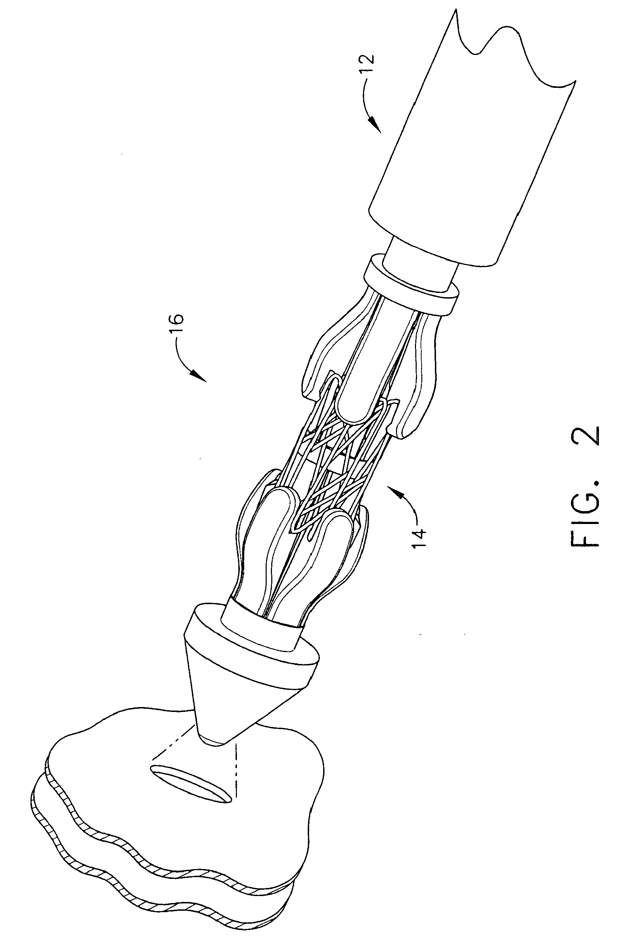

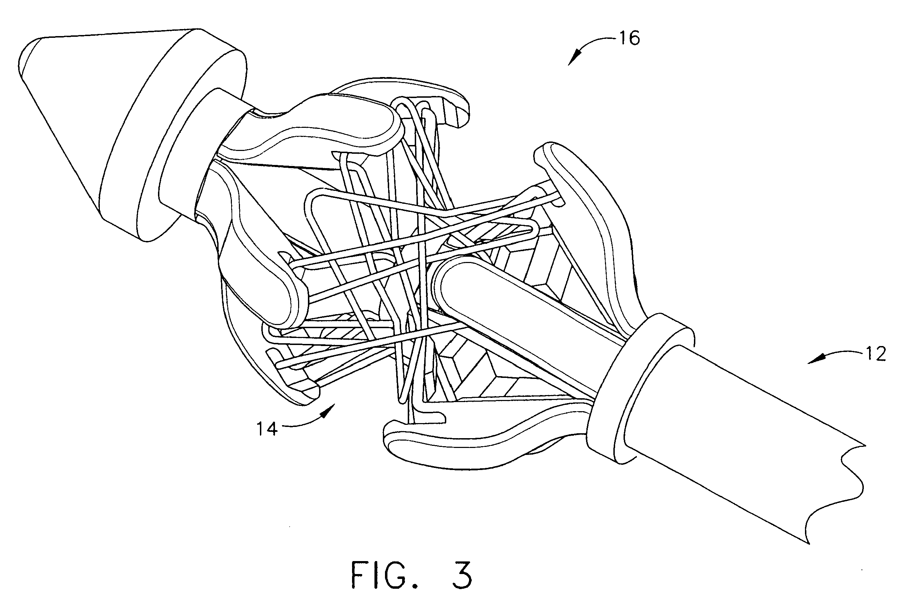

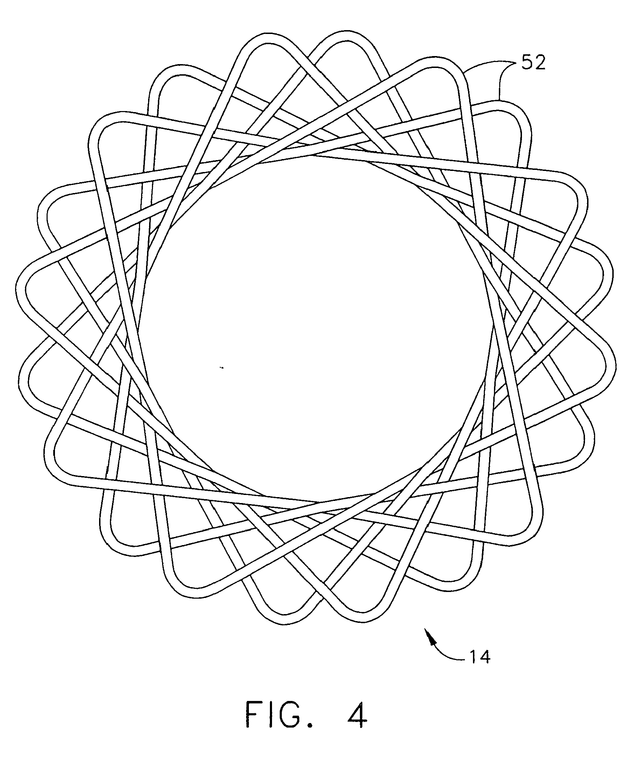

Turning to the Drawings, wherein like numerals denote like components throughout the several views, FIG. 1 depicts an applier 10 that is operable to deploy and actuate an anastomotic ring device 14 (not pictured in FIG. 1) from a generally cylindrical shape to one having properties of a hollow rivet, or ring, capable of forming an anastomotic attachment at an anastomosis target site, such as in a bariatric gastric bypass of a morbidly obese patient. FIG. 2 depicts another applier 12. It will be appreciated that appliers 10, 12 may be used in a variety of ways, including but not limited to laparoscopically or endoscopically. Applier 12 is shown in FIG. 2 with an anastomotic ring 14 on a deployment mechanism 16. In FIG. 2, anastomotic ring 14 is shown in the compressed, cylindrically-shaped position. In FIG. 3, deployment mechanism 16 of applier 12 has moved anastomotic ring 14 to the actuated, hollow rivet-shaped position. FIG. 4 is a close-up view of anastomotic ring 14 in the actuated position. Anastomotic ring 14 may comprise a shape memory effect (SME) material, such as nitinol by way of example only, that further assists in actuation to an engaging hollow rivet shape. Other suitable anastomotic ring 14 materials will be apparent to those of ordinary skill in the art. An exemplary anastomotic ring 14 is described in detail in U.S. Patent Application Publ. No. US 2003/0032967 to Park et al.

It will be appreciated that the terms "proximal" and "distal" are used herein with reference to a clinician gripping a handle of applier 10. It will be further appreciated that for convenience and clarity, spatial terms such as "right'', "left", "vertical" and "horizontal'' are used herein with respect to the drawings. However, surgical instruments are used in many orientations and positions, and these terms are not intended to be limiting and absolute. In addition, aspects of the invention have application to surgical procedures performed endoscopically and laparoscopically, as well as an open procedure or other procedures. Use herein of one of these or similar terms should not be construed to limit the present invention for use in only one category of surgical procedure.

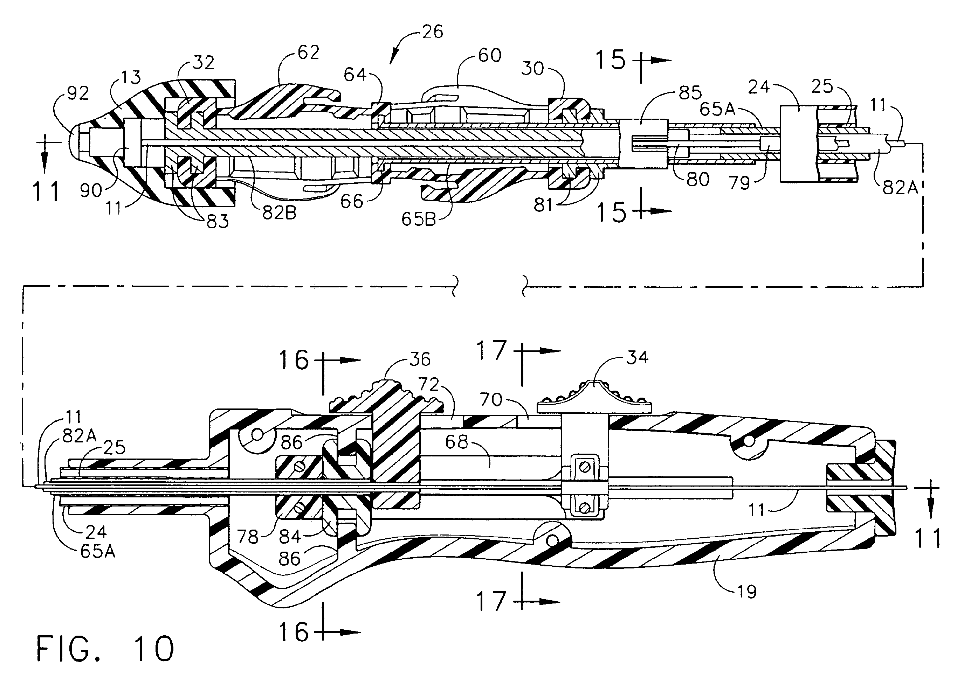

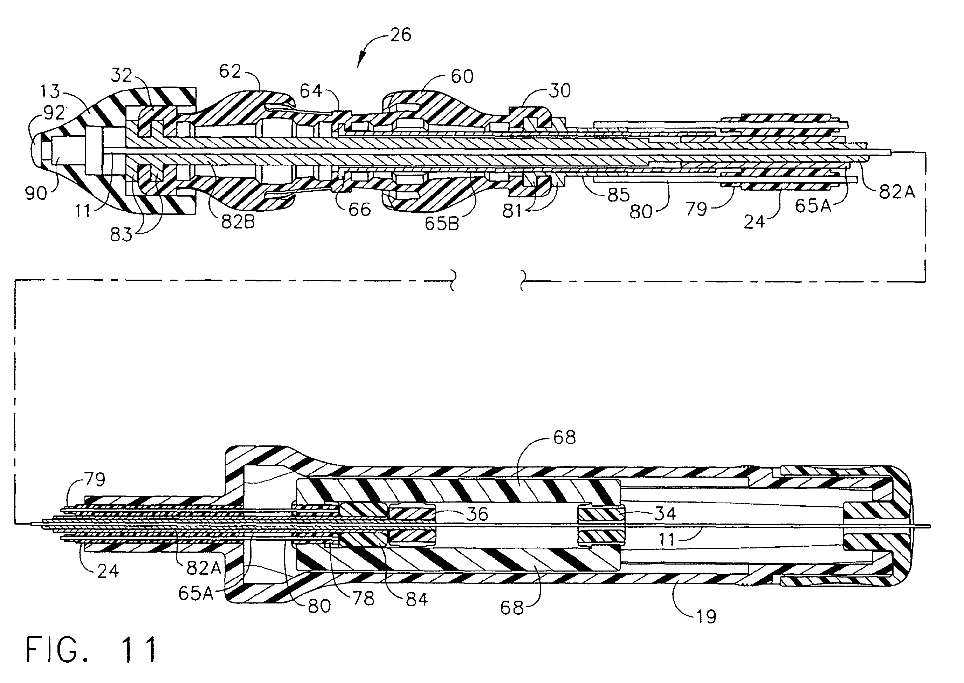

Referring now to FIGS. 1 and 5-15, applier 10 of the present example has a shaft 15 comprising a tubular sheath 24. A handle 19 is positioned at the proximal end of shaft 15, while a ring deployment mechanism 26 is positioned at the distal end of shaft 15. A tip 13 is positioned at the distal end of ring deployment mechanism 26. Applier 10 further comprises an imaging element 11. Imaging element I 1 is coupled with a camera 90 having a lens 92. Camera 90 and lens 92 are positioned in tip 13. Camera 90 may be a CCD camera, a CMOS camera, or any other imaging device. Imaging element 11, camera 90, and lens 92 are configured to provide visualization through tip. Any suitable device, examples of which will be apparent to those of ordinary skill in the art, may be coupled with the proximal end of imaging element 11 for viewing images communicated through imaging element 11. Other suitable configurations for permitting imaging element 11 to capture a view will be apparent to those of ordinary skill in the art. In one embodiment, imaging element I I comprises one or more imaging fibers. In one embodiment, illumination is provided at the distal end of imaging element I 1 by illumination fibers (not pictured) that run adjacent imaging element 11. It will be appreciated that such illumination may aid in the capture of images by imaging element 11 during use of applier 10. Suitable configurations of illumination fibers will be apparent to those of ordinary skill in the art. Of course, illumination may be provided by a variety of alternative means, devices, methods, and/or configurations, or may be eliminated.

In the present example, ring deployment mechanism 26 comprises a plurality of proximal fingers 60 connected to a proximal ring 30; and a plurality of distal fingers 62 connected to a distal ring 32. A stationary mid-ring 64 is longitudinally positioned between proximal ring 30 and distal ring 32. Both proximal fingers 60 and distal fingers 62 are in a double-hinged relationship with stationary mid-ring 64 of ring deployment mechanism 26. Proximal ring 30 is configured to slide distally toward mid-ring 64, causing proximal fingers 60 to actuate outwardly relative to shaft 15. Mid-ring 64 is held stationary by a stationary distal ground tube portion 65B, as will be described below. Likewise, distal ring 32 is configured to slide proximally toward mid-ring 64, causing distal fingers 62 to actuate outwardly relative to shaft 15. Fingers 60, 62 are configured to hold an anastomotic ring 14 by engaging petals 52 prior to and during deployment of the anastomotic ring 14, and release petals 52 upon deployment of the anastomotic ring 14.

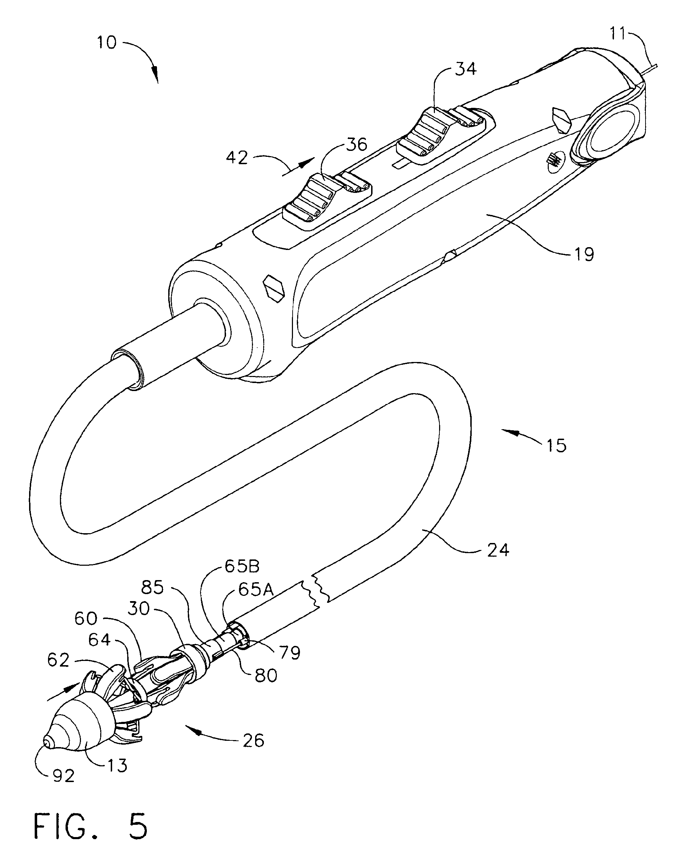

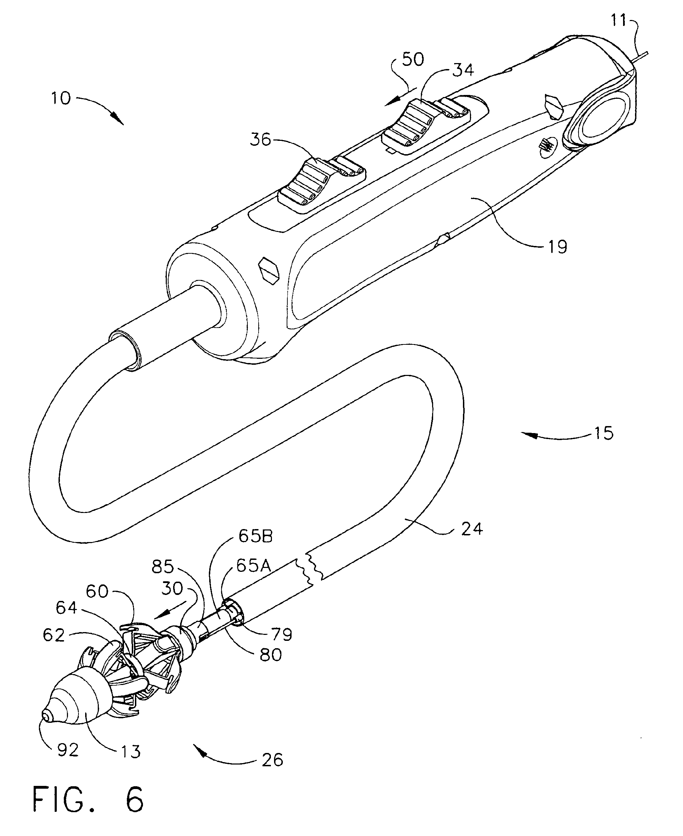

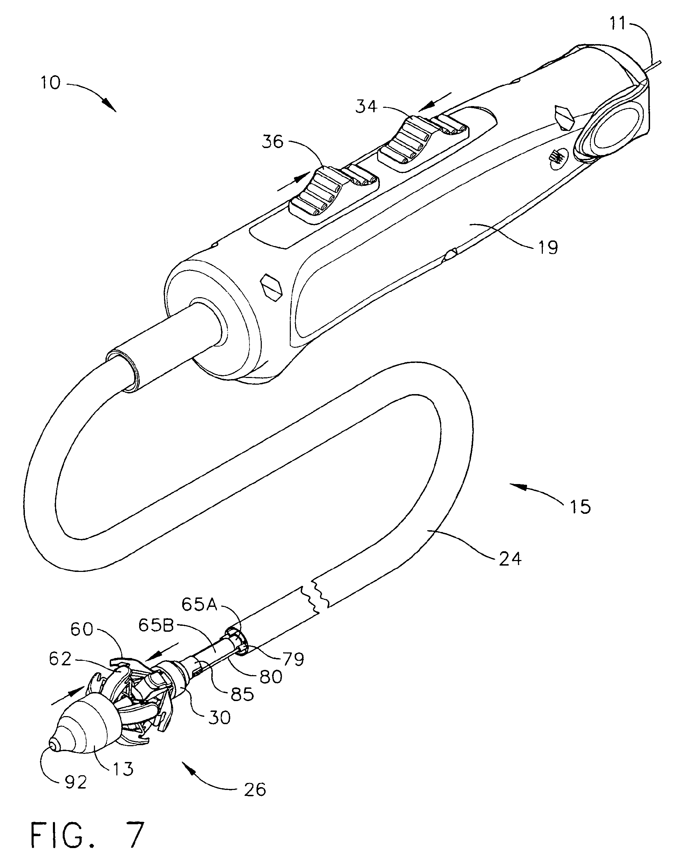

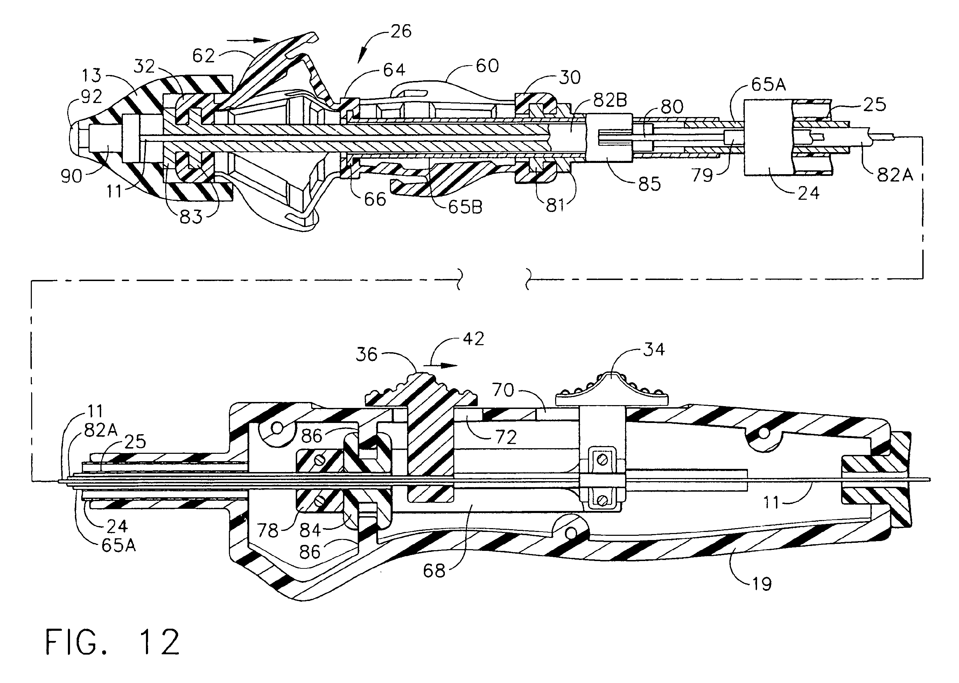

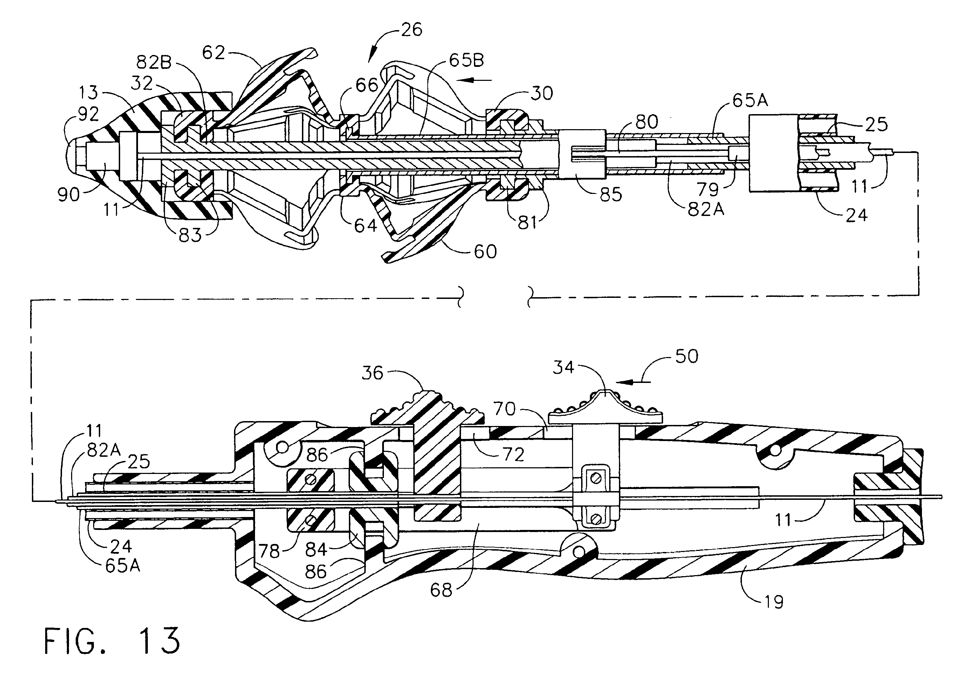

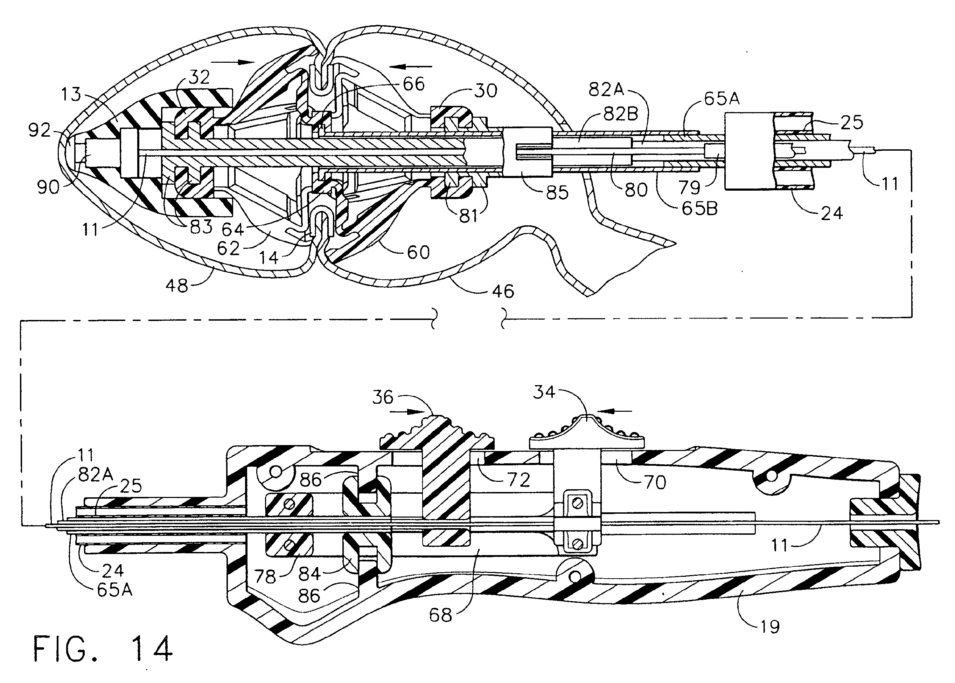

Applier 10 further comprises a pair of deployment actuators 34, 36. As described in more detail below, first deployment actuator 34 is operable to actuate proximal fingers 60 of ring deployment mechanism 26 by advancing proximal ring 30 distally; and second deployment actuator 36 is operable to actuate distal fingers 62 by pulling distal ring 32 proximally. In FIGS. 5 and 12, distal fingers 62 are shown in a partially actuated position for partially deploying a distal portion of an anastomotic ring 14. Arrow 42 depicts actuating motion of second actuator 36. In FIGS. 6 and 13, proximal fingers 60 are shown in a partially actuated position for partially deploying a proximal portion of anastomotic ring 14 to partially complete an anastomotic attachment between proximate tissue walls 46, 48. Arrow 50 depicts the actuating motion of first actuator 34. FIGS. 7 and 14 show fingers 60, 62 in a fully actuated position, effecting deployment of anastomotic ring 14. It will be appreciated that any suitable alternative(s) to ring deployment mechanism 26 and/or deployment actuators 34, 36 may be used.

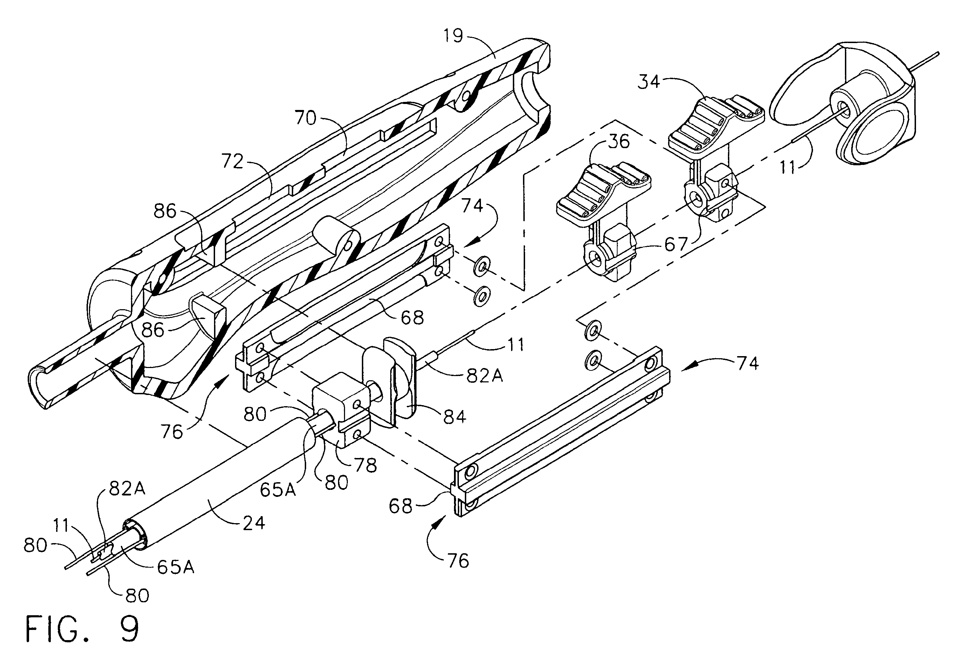

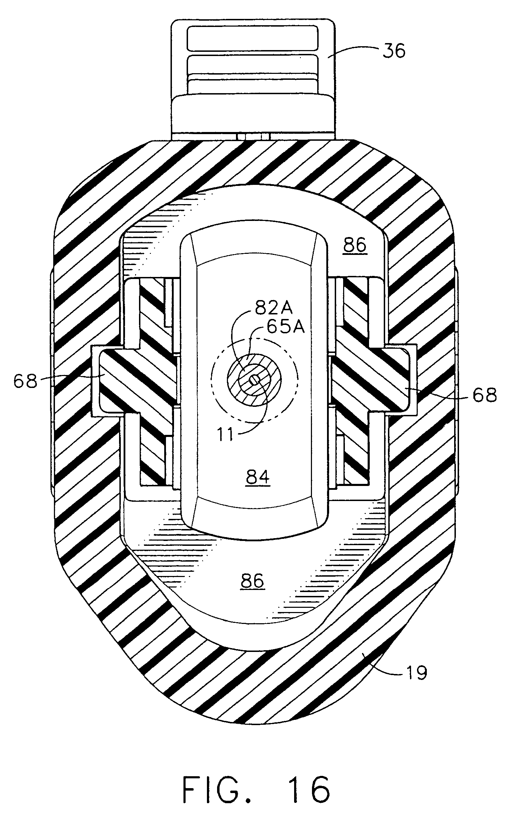

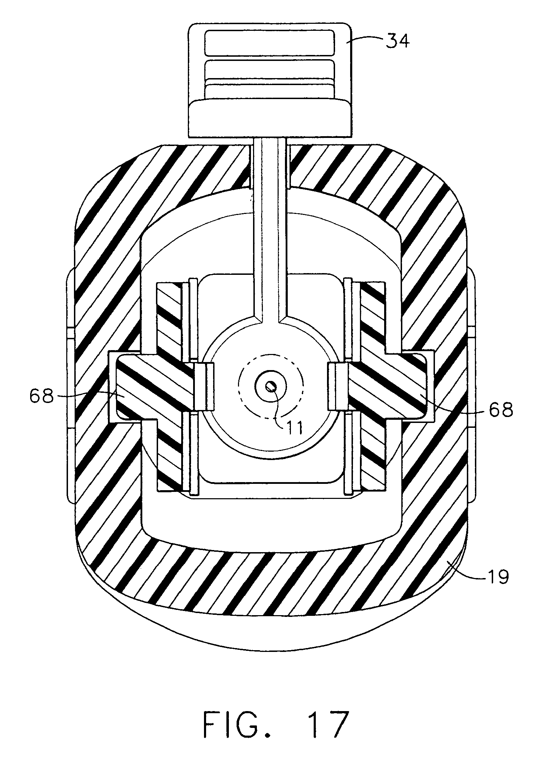

As stated above, first deployment actuator 34 of the present example is operable to control proximal fingers 60; and second deployment actuator 36 is operable to control distal fingers 62. Referring to FIGS. 9 and 16-17, first and second ring deployment actuators 34, 36 each have a pair of grooves 67 that are configured to slide on a track 68 of handle 19. The range of first actuator 34 is limited by the width of a slot 70, while the range of second actuator 36 is limited by the width of a slot 72. In the present example, first actuator 34 is fixedly attached to a proximal portion 74 of track 68. Track 68 is slideable within handle 19. A distal portion 76 of track 68 is fixedly attached to a slider 78. Slider 78 is fixedly connected to a pair of push cables 80. Longitudinal motion of first actuator 34 is thereby operable to cause corresponding longitudinal motion of track 68, slider 78, and push cables 80. Other suitable relationships between these components, as well as alternative components and configurations, will be apparent to those of ordinary skill in the art.

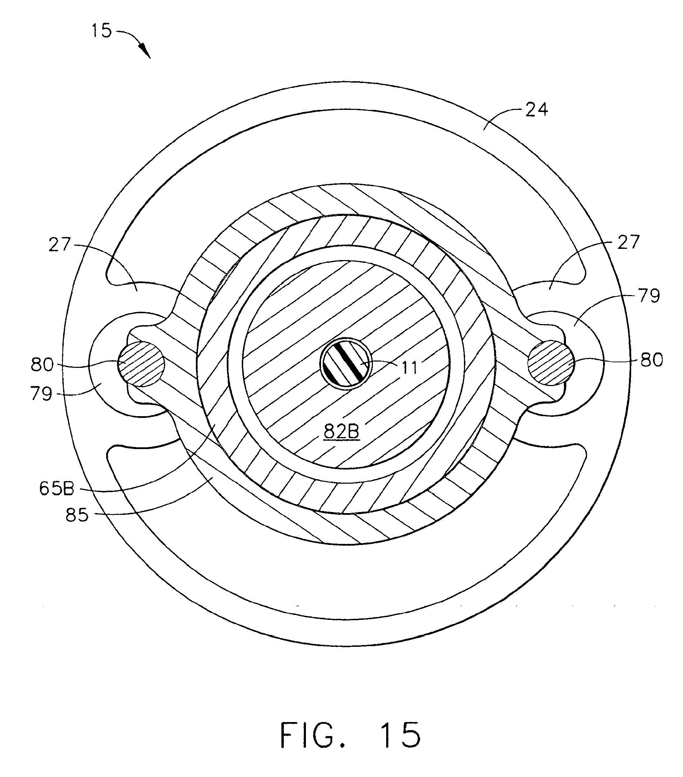

Referring to FIG. 15, push cables 80 are each positioned within a respective cable sheath 79. Each cable sheath 79 extends longitudinally through a respective cable conduit 27 in shaft 15. In the present example, cable conduits 27 are formed in sheath 24.

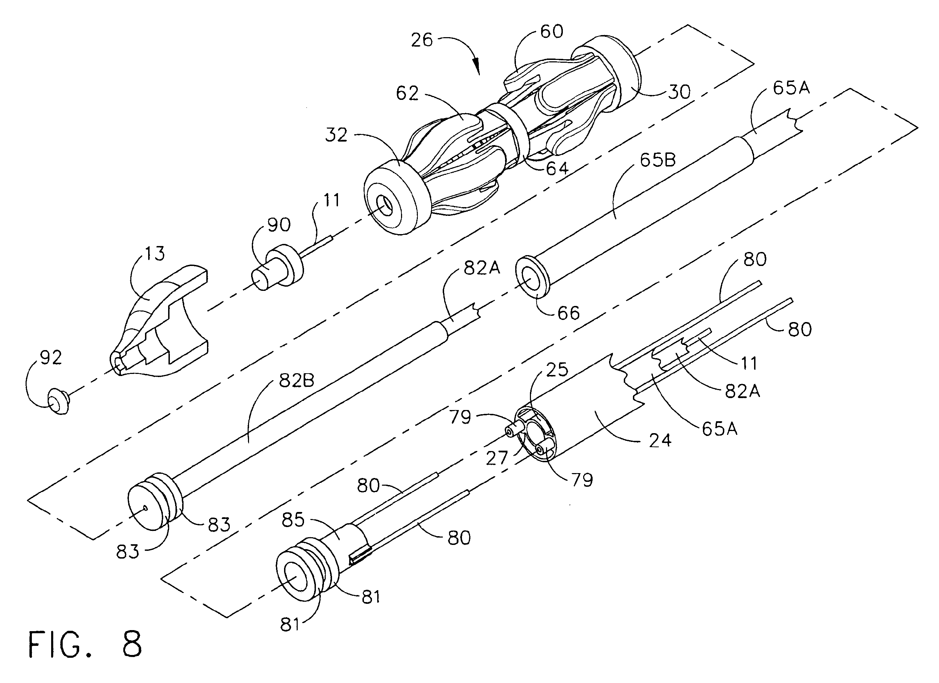

Referring to FIGS. 8 and 10-15, the distal end of each push cable 80 is fixedly secured to the proximal end of a push tube 85. The distal end of push tube 85 has a pair of flanges 81, which are configured to engage with proximal ring 30 of ring deployment mechanism 26. Accordingly, push tube 85 is operable to communicate longitudinal motion to proximal ring 30, thereby actuating or deactuating proximal fingers 60. Because of engagement between push cables 80 and push tube 85, such longitudinal motion may be provided by actuation of first actuator 34. Of course, any other suitable components or configurations may be used.

Shaft 15 further comprises a proximal ground tube portion 65A extending longitudinally therethrough. The proximal end of proximal ground tube portion 65A is fixedly attached to anchor member 84. Anchor member 84 is configured to engage with bosses 86, which are integral with handle 19. Accordingly, in the present example, anchor member 84 and bosses 86 are configured to prevent relative movement between proximal ground tube portion 65A and handle 19. Proximal ground tube portion 65A extends longitudinally through ground tube conduit 25, which is formed in sheath 24 adjacent to and between cable conduits 27. A distal ground tube portion 65B is fixedly secured to proximal ground tube portion 65A. The distal end of distal ground tube portion 65B has a flange 66, which is configured to engage mid-ring 64 of ring deployment mechanism 26. Ground tube portions 65A, 65B thus prevent longitudinal movement of mid-ring 64 relative to handle 19. It will be appreciated that any other suitable components or configurations may be used.

Second actuator 36 is fixedly secured to a proximal inner tube portion 82A, which extends longitudinally through proximal ground tube portion 65A. A distal inner tube portion 82B is fixedly secured to proximal inner tube portion 82A. Distal inner tube portion 82B extends longitudinally through distal ground tube portion 65B. The distal end of distal inner tube portion 82B has a pair of flanges 83, which are configured to engage with distal ring 32 of ring deployment mechanism.
Accordingly, distal inner tube portion 82B is operable to communicate longitudinal motion to distal ring 32, thereby actuating or deactuating distal fingers 62. Because of engagement between distal inner tube portion 82B and proximal inner tube portion 82A, such longitudinal motion may be provided by actuation of second actuator 36. Of course, any other suitable components or configurations may be used.

It should be noted that although second actuator 36 is configured to slide on track 68 in the present example, second actuator 36 is not statically attached to track 68. Therefore, longitudinal movement of track 68 caused by motion of first actuator 34 does not cause longitudinal movement of second actuator 36. Of course, handle 19 and components thereof may be configured in any other suitable way. By way of example only, first actuator 34 may be configured to control actuation of distal fingers 62, and second actuator 36 may be configured to control actuation of proximal fingers 60. Still other suitable alternative configurations will be apparent to those of ordinary skill in the art.

It will be appreciated that applier 10 of the present example may be used to deploy an anastomotic ring 14 without the involvement of a nonfunctional enterotomy. For instance, applier 10 may be configured such that tip 13, ring deployment mechanism 26, and shaft 15 may be inserted down the esophagus of a patient. Accordingly, shaft 15 may be sized (e.g., as to diameter and length) to reach an anastomosis site transorally, via the esophagus. Particularly when imaging element 11, camera 90, and lens 92 are included in applier 10, such use may eliminate the need for at least one trocar port in the abdomen or elsewhere in the patient. Of course, applier 10 may be used in any other suitable way, including but not limited to use through a nonfunctional enterotomy or use in an open procedure.

In the present example, shaft 15 is flexible. However, it will be appreciated that shaft 15, including components thereof, may have any other properties, including but not limited to malleability, rigidity, resilience, other properties, or combinations thereof. It will also be appreciated that components of applier 10 may have any suitable dimensions. By way of example only, tip 13, ring deployment mechanism 26, and/or shaft may have a maximum outer diameter between approximately 12 and 18 mm. Of course, any other dimensions may be used.

In another embodiment, one or more tubes 82A, 82B, 65A, 65B, and/or 85 are eliminated, and cables are substituted therefor. For instance, a pull cable may be substituted for proximal inner tube portion 82A. Cables may be individually sheathed and/or bundled together, or have any other components or configurations. It will be appreciated that, by providing sheaths about cables, buckling of the cables may be prevented. Of course, sheaths may provide other advantages over sheathless cables, or may provide no advantages at all. As with several other components, sheaths, including but not limited to cable sheaths 79, are optional. For instance, cable sheath conduits 27 may be configured to provide sheathing for push cables 80.

In yet another embodiment, shaft 15 comprises one or more working channels. By way of example only, such channels may be used to introduce micro forceps or any other device. Where micro forceps are included, the same may be used to grasp tissue to be cut or anastomosed. Other variations will be apparent to those of ordinary skill in the art.

In still another embodiment, proximal ground tube portion 65A is eliminated, and ground tube conduit 25 provides grounding for mid-ring 64. In one version of this embodiment, the proximal end of ground tube conduit 25 extends into handle 19 further than other portions of sheath 24, and is fixedly secured to anchor member 84. In this alternate version, the distal end of ground tube conduit 25 is fixedly secured to distal ground tube portion 65B. Any other variation of, substitute for, or supplement to ground tube 65A, 65B may be used.

In use, applier 10 may be inserted adjacent an anastomotic opening in proximate tissue walls 46, 48. Imaging element 11, camera 90, and lens 92 may be used to capture a view of the anastomosis site, such as to properly position applier 10. As shown in FIGS. 5 and 12, second actuator slider 36 may be partially actuated to partially actuate distal fingers 62, thereby partially deploying a distal portion of anastomotic ring 14. As shown in FIGS. 6 and 13, first actuator slider 34 may be partially actuated to partially actuate proximal fingers 60, thereby partially deploying a proximal portion of anastomotic ring 14. The surgeon may then confirm proper positioning of applier 10, such as through tactile feedback or through any other technique. As shown in FIGS. 7 and 14, first and second actuator sliders 34, 36 may be fully actuated to fully actuate ring deployment mechanism 26, thereby completing deployment of anastomotic ring 14 to effect an anastomosis. After anastomotic ring 14 has been deployed, the above steps may be reversed, and applier 10 may be withdrawn. Other variations of use of applier 10 will be apparent to those of ordinary skill in the art.

While applier 10 has been described as being operable to deploy an anastomotic ring 14, it will be appreciated that applier 10 may have a variety of other uses. By way of example only, where an anastomotic ring 14 has already been deployed, applier 10 may be used to compress or otherwise modify or manipulate the deployed anastomotic ring 14. Still other uses will be apparent to those of ordinary skill in the art.

Having shown and described various embodiments and concepts of the invention, further adaptations of the methods and systems described herein can be accomplished by appropriate modifications by one of ordinary skill in the art without departing from the scope of the invention. Several of such potential alternatives, modifications, and variations have been mentioned, and others will be apparent to those skilled in the art in light of the foregoing teachings. Accordingly, the invention is intended to embrace all such alternatives, modifications and variations as may fall within the spirit and scope of the appended claims and is understood not to be limited to the details of structure and operation shown and described in the specification and drawings. Additional advantages may readily appear to those skilled in the art.

## Claims

1. A surgical instrument for deploying an anastomotic ring device at an anastomosis site, comprising:
(a) a ring deployment mechanism, wherein the ring deployment mechanism is configured to receive and deploy an anastomotic ring;
(b) an elongate shaft comprising a plurality of actuation members, wherein each of the actuation members is operable to communicate one or more actuating forces to the ring deployment mechanism, wherein the shaft is flexible; and
(c) one or more actuators, where in each of the one or more actuators is operable to communicate one or more actuating forces to at least one of the actuation members.

2. The surgical instrument of Claim 1, wherein the shaft further comprises an imaging element.

3. The surgical instrument of Claim 2, further comprising a tip positioned distally relative to the ring deployment mechanism, wherein the tip comprises a lens in communication with the imaging element.

4. The surgical instrument of Claim 1, wherein at least one of the one or more actuator members comprises a slider.

5. The surgical instrument of Claim 1, further comprising a handle, wherein at least one of the one or more actuator members is positioned on the handle.

6. The surgical instrument of Claim 1, wherein at least a portion of the actuation members comprises one or more tubes.

7. The surgical instrument of Claim 1, wherein at least a portion of the actuation members comprises one or more cables.

8. The surgical instrument of Claim 1, wherein at least a portion of the actuation members comprises a combination of tubes and cables.

9. The surgical instrument of Claim 1, wherein the ring deployment mechanism comprises a plurality of fingers.

10. The surgical instrument of Claim 9, wherein the plurality of fingers comprises a set of distal fingers and a set of proximal fingers.

11. A surgical instrument for deploying an anastomotic ring device at an anastomosis site, the instrument comprising:
(a) a handle having one or more actuators, wherein each of the one or more actuators is configured to receive user input to provide one or more actuating forces;
(b) an elongate shaft having a first end and a second end, wherein the handle is connected to the first end of the elongate shaft, wherein the elongate shaft comprises one or more actuation members, wherein the one or more actuation members are in communication with the one or more actuators, wherein the shaft and the one or more actuation members are flexible; and
(c) a ring deployment mechanism positioned adjacent the second end of the shaft, wherein the ring deployment mechanism is configured to receive an anastomotic ring device, wherein the one or more actuation members are configured to communicate the one or more actuating forces to the ring deployment mechanism, wherein the ring deployment mechanism is operable to deploy the anastomotic ring device in response to at least one of the one or more actuating forces.
